# EUROPÄISCHE PATENTSCHRIFT

(11) **EP 3 958 815 B1**
(45) Veröffentlichungstag und Bekanntmachung des Hinweises auf die Patenterteilung: **27.08.2025**
(21) Anmeldenummer: 20721731.6
(22) Anmeldetag: 27.03.2020
(51) Int. Cl.: A61H 31/00, A61N 1/39

(54) **GERÄT ZUR UNTERSTÜTZUNG EINES ERSTHELFERS BEI EINER HERZ-LUNGEN-WIEDERBELEBUNG**
DEVICE FOR ASSISTING A FIRST AIDER WITH A CARDIOPULMONARY RESUSCITATION
APPAREIL POUR ASSISTER UN SECOURISTE LORS D'UNE RÉANIMATION CARDIO-PULMONAIRE

(30) Priorität: 23.04.2019 DE 102019110455; 23.07.2019 DE 102019119855
(43) Veröffentlichungstag der Anmeldung: 02.03.2022
(73) Patentinhaber: SmartResQ GmbH, 73770 Denkendorf (DE)
(72) Erfinder: MÜLLER, Michael, 79117 Freiburg (DE); ROTH, Matthias, 79111 Freiburg (DE); SCHORLING, Per, 5700 Svendborg (DK)
(74) Vertreter: Werner, André
(86) Internationale Anmeldenummer: PCT/IB2020/052915
(87) Internationale Veröffentlichungsnummer: WO 2020/217115

(56) Entgegenhaltungen:
- EP-A1- 2 228 097
- EP-A1- 2 653 146
- EP-B1- 2 228 097
- WO-A1-2013/128306
- WO-A1-2019/015727
- WO-A1-2020/006355
- CN-A- 106 902 462
- US-A1- 2016 287 470
- US-A1- 2017 259 054
- US-A1- 2018 169 426

## Beschreibung

Die Erfindung betrifft ein Gerät zur Erfassung von Daten und zur Anleitung eines Helfers bei der Durchführung von Erste-Hilfe-Maßnahmen zur Reanimation eines von einem Kreislaufstillstand betroffenen Patienten gemäß Oberbegriff des Anspruchs 1.

Überleben nach plötzlichem Herztod ist nur möglich, wenn innerhalb der ersten Minuten, also in der Regel noch vor dem Eintreffen des Rettungsdienstes eine Herzdruckmassage durchgeführt wird und diese entsprechend der jeweils aktuellen Empfehlungen der Leitlinien erfolgt. Weiterhin ist die Beatmung des Patienten nötig. Die Abgabe eines Defibrillationsschocks innerhalb der ersten Minute bei Vorliegen eines Kammerflimmerns oder einer ventrikulären Tachykardie erhöht zudem die Überlebenswahrscheinlichkeit auf ca. 90%.

Beispielsweise leiten in Deutschland nur in etwa 40% der Notfälle Laienhelfer die wichtigen Maßnahmen der Wiederbelebung (vor allem Herzdruckmassage) ein. Die Qualität der Herzdruckmassage ist nach Schulung und praktischem Training gut, nimmt aber bereits nach drei bis sechs Monaten wieder erheblich ab. Zudem sinkt auch während einer Wiederbelebung bereits nach zwei Minuten die Qualität der Herzdruckmassage erheblich ab. Auch löst das Durchführen einer Herzdruckmassage zumindest beim Laienhelfer erhebliche Stressreaktionen aus, in der eine Anleitung des Helfers die Qualität der Herzdruckmassage verbessern kann.

Die Überlebenswahrscheinlichkeit eines Patienten mit Kreislaufstillstand hängt wesentlich von den Erste-Hilfe-Maßnahmen des Laienhelfers ab, der mit der Situation jedoch häufig überfordert und selten ausreichend trainiert ist. Selbst bei geschulten Laienhelfern ist die Qualität der Maßnahmen oft unzureichend. Aus diesem Grund sind den Helfer bei der Reanimation unterstützende Systeme sehr hilfreich.

Aus EP 1 128 795 B1 ist ein System zum Messen und Veranlassen von Brustkompressionen bekannt. Es umfasst einen mobilen CPR-Kompressionsmonitor (CPR - Cardiopulmonale Reanimation, Herz-Lungen-Wiederbelebung) zur Überwachung der Thoraxkompressionen bei einer Reanimation einer von einem Kreislaufstillstand betroffenen Person. Das Gerät wird auf der Hand des Helfers oder auf dem Patienten abgelegt und umfasst Beschleunigungssensoren sowie eine Schnittstelle zur Datenübertragung. An diese Schnittstelle wird über ein Kabel eine im CPR-Kompressionsmonitor integrierte oder eine eigenständige Auswerteeinheit mit Bildschirm angeschlossen. Dieses System ist für geschultes und erfahrenes medizinisches Personal konzipiert.

WO 2006/104977 A2, EP 2 255 845 A1 und DE 60 2004 002 147 T2 offenbaren ein vielschichtiges professionelles medizinisches System zur Unterstützung eines Ersthelfers, wobei dieser in der Reanimation geschult sein muss. Das System umfasst unter anderem einen Defibrillator und ein mobiles Anzeige- und Steuergerät.

WO 2013/ 128 306 A1 offenbart die Merkmale der Präambel von Anspruch 1 und beschreibt einen automatischen externen Defibrillator, der für den Einsatz im Herznotfall konzipiert ist. Der Defibrillator kann in einem Defibrillator-Modus verwendet werden, indem der Defibrillator einem Patienten eine Elektrotherapie verabreicht. Zusätzlich zu seiner Elektro-Wiederbelebungsfunktion kann der Defibrillator auf der Brust des Patienten eingesetzt werden, um Herz-Lungen-Wiederbelebungs-Kompressionen durchzuführen.

US 2018/169426 A1 offenbart ein Gerät sowie Software und Methodik im Zusammenhang mit einem tragbaren automatisierten externen Defibrillator. Der tragbare Defibrillator interagiert mit einem mobilen Gerät und umfasst zwei oder mehr Herzelektroden, einen Akku und einen Spezialkondensator. Wenn der Defibrillator an einem Patienten mit Herzstillstand angeschlossen wird, kontaktiert er den Rettungsdienst und zeichnet Patienteninformationen auf, die zur Auswertung an einem medizinischen Dienstleister übermittelt werden. Der Defibrillator ist in der Lage, Herzrhythmen zu analysieren, schlägt bei entsprechender Herzrhythmusstörung die Verabreichung eines oder mehrerer Schocks vor und weist den Benutzer in die richtige CPR-Technik ein.

EP 2 653 146 A1 beschreibt ein Korrekturanweisungssystem für die Herzdruckmassage unter Zuhilfenahme eines automatisierten externen Defibrillators. Dieser umfasst einen Sensor, der Kompressionsmessdaten von CPR-Brustkompressionen erhält, sowie ein Steuersystem mit einem Mikroprozessor, der so programmiert ist, dass er eine nichtparametrische, informationstheoretische Analyse der Kompressionsmessdaten durchführt.

EP 2 228 097 A1 offenbart einen Defibrillator mit einem Thoraxkompression-Feedbackmittel, der hinsichtlich Form und Größe so ausgebildet ist, dass er direkt auf der Brust eines Patienten platziert werden kann. Das Thoraxkompression-Feedbackmittel dient zum Ermitteln von quantitativen und qualitativen Daten über eine an dem Patienten durchgeführte Thoraxkompression.

US 2016/287470 A1 beschreibt ein medizinisches Gerät, umfassend eine Dienstkomponente zur Verwendung bei der Erkennung von Patientendaten, ein Pflegeprotokollmodul zur Gesundheitsversorgung eines Patienten und ein Ressourcenmodul zur Verwaltung des Zugriffs auf die Dienstkomponente und zur Beantwortung einer Anfrage durch Verwaltung der Dienstkomponente.

CN 106 902 462 A offenbart ein tragbares Gerät zur automatischen Herz-Lungen-Wiederbelebung, umfassend eine Hülle, ein in der Hülle angeordnetes Defibrillationsmodul zur Durchführung der Herz-Lungen-Wiederbelebung und ein in der Hülle angeordnetes Beatmungshilfemodul zur Beatmungsunterstützung.

WO 2019/015727 A1 beschreibt eine Sensoreinrichtung zur Erfassung von Daten bei der Durchführung von Ersthilfsmaßnahmen zur Reanimation einer von einem Kreislaufstillstand betroffenen Person. Die Sensoreinrichtung wird auf dem Brustkorb des Patienten mittels einer Positionierungshilfsvorrichtung positioniert und mittels eines Befestigungsmittels fixiert. Über einen Bewegungssensor sind die Parameter der Thoraxkompressionen, zum Beispiel die Eindrucktiefe und die Kompressionsfrequenz, erfassbar. Eine Schnittstelle zur Datenübertragung erlaubt die Kommunikation mit einem mobilen Endgerät.

US 2017 / 0 259 054 A1 offenbart eine Elektrodenanordnung zur Verwendung mit einem Defibrillator, wobei die Elektrodenanordnung mindestens eine Elektrode mit einer ersten Oberfläche, die entweder an einem pädiatrischen Patienten oder einem erwachsenen Patienten befestigt werden kann, und einer zweiten Oberfläche umfasst, wobei der Großteil der zweiten Oberfläche bebilderte Anweisungen zur Verwendung der Elektrodenbaugruppe aufweist.

WO 2020/006355 A1 beschreibt ein für die Herz-Lungen-Wiederbelebung vorgesehenes System aus einem Brustkompressionsgerät, zum Beispiel einem Defibrillator, und einem Computergerät, wobei das Computergerät konfiguriert ist, Informationen über einen Patienten zu erhalten, der wegen eines Herz-Lungen-Stillstands behandelt wird, und Befehle an das Brustkompressionsgerät zu senden.

Desgleichen beschreibt EP 1 858 472 B1 ein zwar mobiles, doch sehr komplexes medizinisches System zur Unterstützung eines Helfers bei der Reanimation, das auch einen Defibrillator umfasst. Es ist vorgesehen, das Gerät an wenigen zentralen Orten mit hohem Menschenaufkommen zu stationieren, damit Ersthelfer einen schnellen Zugriff darauf erhalten. Allerdings ist auch dieses Gerät nur von geschulten Helfern einigermaßen sinnvoll einsetzbar.

Ein weiterer Nachteil der o.g. Geräte ist ihre Größe und ihr Gewicht, die ein tagtägliches Mitführen unmöglich machen, sodass diese Geräte z. B. an zentralen Plätzen vorgehalten werden. Allerdings weiß in einem Notfall ein Ersthelfer oft nicht, wo er ein derartiges Erste-Hilfe-Gerät finden kann bzw. es verstreichen wertvolle Minuten, bis das Gerät herangeschafft wird. Auch ist ein (Erst-)Helfer häufig überfordert, insbesondere wenn er vor Eintritt des Notfalls nicht ausreichend an dem jeweiligen Gerät trainiert wurde.

Speziell bei Systemen mit automatischem Defibrillator hat sich zudem gezeigt, dass bis zum Beginn der ersten Herzdruckmassage vergleichsweise viel Zeit - im Mittel knapp zwei Minuten - aufgrund vorbereitender Maßnahmen verloren geht. Dies steht jedoch im Widerspruch zu den Empfehlungen für wiederbelebende Maßnahmen, wonach schnellstmöglich, d. h. nach weniger als 30 Sekunden, eine Herzdruckmassage durchzuführen ist.

Der Erfindung liegt die Aufgabe zugrunde, ein Gerät zur Unterstützung eines Ersthelfers bei einer Herz-Lungen-Wiederbelebung bereitzustellen, das eine Sensoreinrichtung zur Erfassung von Daten bei der Durchführung von Ersthilfsmaßnahmen zur Reanimation umfasst, wobei das Gerät so klein und kompakt sein soll, dass es von jedem hilfswilligen Laien oder Ersthelfer stets mitgeführt werden kann, und wobei die Handhabung absolut intuitiv sein soll, sodass eine Anwendung zur unverzüglichen Reanimation auch in Stresssituationen von einem ungeschulten Laienhelfer, der keine medizinischen Vorkenntnisse besitzt, ermöglicht ist.

Die Aufgabe wird durch ein Gerät zur Unterstützung eines Ersthelfers mit den kennzeichnenden Merkmalen nach Patentanspruch 1 gelöst; zweckmäßige Ausgestaltungen der Erfindung finden sich in den Unteransprüchen.

Gemäß der Erfindung wird ein Gerät zur Unterstützung eines Ersthelfers bei einer Wiederbelebung bereitgestellt, das aufgrund seiner Abmessungen und seines Gewichts tag-täglich, z. B. in einer Handtasche, mitführbar ist. Das Gerät umfasst eine Sensoreinrichtung, beinhaltend Sensoren zur Erfassung der bei der Herzdruckmassage relevanten Daten, sowie Elektroden zur Messung der EKG-Daten des Patienten und zur Abgabe von Defibrillationsschocks.

Das Gerät ist konzipiert zur Unterstützung von Ersthelfern, insbesondere aber auch von unzureichend ausgebildeten Laienhelfern, bei der Reanimation von Patienten mit einem Kreislaufstillstand.

Das erfindungsgemäße Gerät umfasst ein vorzugsweise starres Transportgehäuse, in welchem die Sensoreinrichtung, Kabel und mindestens zwei Elektroden verstaut sind. Dieses Transportgehäuse kann als eine zweiteilige, flache Box ausgebildet sein, beispielsweise in Form eines Schlüsselanhängers. Erfindungsgemäß liegen die äußeren Abmessungen innerhalb von 10 cm x 10 cm x 3 cm.

Das Transportgehäuse ist aufgrund seiner Materialauswahl und/oder seines Aufbaus als zumindest spritzwassergeschützter Behälter ausgelegt, der eine, z. B. vor Schmutz, geschützte Lagerung seines Inhaltes über wenigstens vier Jahre erlaubt.

Die Sensoreinrichtung umfasst einen Bewegungssensor, einen Speicher für elektrische Energie, nachfolgend Batterie genannt, einen Mikroprozessor, einen Hochspannungsspeicher (z. B. in Form eines Kondensators) für einen Defibrillations-Impuls, Anschlüsse für die Elektroden, eine Schnittstelle zum Senden und/oder Empfangen von Daten und/oder Befehlen sowie an seiner Außenhülle ein Haftmittel zur Befestigung der Sensoreinrichtung auf dem Brustkorb des Patienten.

Die Elektroden weisen auf einer Seite eine mit einer abziehbaren Schutzfolie abgedeckte Klebefläche auf. Außerdem weisen die Elektroden Knickfalze auf, sodass sie während der Lagerung im Transportgehäuse zusammengefaltet sein können. Mittels der Elektroden werden elektrische Ströme/Spannungen erfasst, z. B. zur Aufzeichnung eines Elektrokardiogramms (EKG), und/oder ein Defibrillationsschock auf den Patienten übertragen. Mittels der Kabel sind die Elektroden mit der Sensoreinrichtung verbindbar.

Der Mikroprozessor ist derart ausgestaltet, dass die mittels der Sensoren und Elektroden erfassten Daten auswertbar und in einer von einem über die Schnittstelle mit der Sensoreinrichtung drahtlos oder mittels Kabel verbundenen mobilen Endgerät darstellbaren bzw. weiterverarbeitbaren Form aufbereitbar sind. Das mit der Sensoreinrichtung kommunizierende mobile Endgerät kann jede Art von handelsüblichem tragbarem Klein- oder Kleinstcomputer, zum Beispiel ein Smartphone, ein Phablet, ein Tablet oder eine Smartwatch, sein, wobei das mobile Endgerät selbst kein Bestandteil der Erfindung ist.

Das mit der Sensoreinrichtung verbundene mobile Endgerät empfängt Daten und gibt dem Ersthelfer über eine auf dem Endgerät zu installierende Anwendungssoftware Anweisungen zur optimalen Reanimation. Die auf dem Endgerät laufende Software kann Daten von den Elektroden und der Sensoreinrichtung kombinieren, um valide Ergebnisse zu erzielen. So können beispielsweise Impedanzen (von den Elektroden) mit Bewegungsdaten des Lage- und/oder Bewegungssensors zusammen eine valide Beurteilung der Spontanatmung bzw. Beatmung durch den Helfer ermöglichen. Somit erhält der Ersthelfer Rückmeldungen über die Qualität seiner durchgeführten Maßnahmen, insbesondere der Herzdruckmassage, und einen Hinweis, falls ein Defibrillationsschock notwendig ist.

In der Sensoreinrichtung wird - entweder ferngesteuert aus dem angeschlossenen Endgerät oder sofort nach dem Einschalten der Sensoreinrichtung - ein Defibrillationsschock vorbereitet, d. h., der z.B. in Form eines Kondensators ausgebildete Hochspannungsspeicher wird aufgeladen. Nach automatisierter Auslösung durch das mobile Endgerät und/oder nach manuellem Auslösen durch den Ersthelfer mittels eines Auslöseknopfes der Sensoreinrichtung und/oder über eine Option der auf dem mobilen Endgerät laufenden Software wird der Strom aus dem Hochspannungsspeicher an den Patienten als Defibrillationsschock abgegeben. Zum Beispiel wird die Auslösung des Schocks automatisiert durch die auf dem mobilen Endgerät laufenden Software vorbereitet, wobei die Auslösung vom Ersthelfer durch Betätigung des Auslöseknopfes der Sensoreinrichtung erst bestätigt werden muss.

Die Batterie sowie der Hochspannungsspeicher sind - aufgrund der Größe der Sensoreinrichtung - dimensioniert, einen Defibrillationsschocks zu generieren. Das Gerät erfüllt zwar nicht die üblichen Anforderungen an einen (Automatisierten) Externen Defibrillator, da die damit nötigen äußeren Abmessungen einem ständigen Mitführen (beispielsweise in der Hand- oder Jackentasche) entgegenstehen würden, aber es kann durch Abgabe zumindest eines Defibrillationsschocks eine erfolgreiche Reanimation ermöglichen, wenn dieser schnell genug erfolgt.

Der Vorteil der Erfindung ist, dass das Gerät im Vergleich zu den aus dem Stand der Technik bekannten medizinischen Geräten zur Unterstützung eines Ersthelfers bei der Reanimation (insbesondere solche mit zusätzlichem Defibrillator) sehr klein und folglich ohne Weiteres transportabel ist. Zudem ist es auf Grund der kleineren Größe auch deutlich kostengünstiger, wodurch es breiter einsatzbar sein wird - insbesondere für jeden zugängig. Da das Gerät aufgrund seiner Größe z.B. im Handschuhfach eines Kraftfahrzeugs mitführbar ist, ist es in Notfallsituationen sofort verfügbar und dementsprechend kann ein eventuell notwendiger Defibrillationsschock innerhalb weniger als 2 Minuten abgegeben werden. Das Gerät ist darauf ausgelegt, bei der Notfallversorgung in den ersten Minuten zu unterstützen. Es kann zwar - falls nötig - einen Defibrillationsschock auslösen, soll jedoch keinesfalls einen Defibrillator ersetzen. Gelegentlich sind im Rahmen der Reanimation auch mehrfache oder gar viele Defibrillationsschocks notwendig, sodass ein zusätzlicher externer Defibrillator zum Einsatz kommen muss, wenn die Reanimation mit dem hier beschriebenen Gerät nicht innerhalb weniger Minuten erfolgreich ist.

Ein weiterer Vorteil ist, dass die nötigen Prozesse - wie das Ableiten der Herzströme, die Analyse und Interpretation der Messdaten, die Anleitung des Ersthelfers bei den Notfallmaßnahmen und (ggf. in Kombination mit einem am Gerät zu betätigenden Schalter) das Auslösen des Defibrillationsschocks - von dem mit der Sensoreinrichtung verbundenen Endgerät übernommen werden. Diese Ausgestaltung der Erfindung ermöglicht es, das Gerät, d. h. die zusätzlich zum bereits vorhandenen mobilen Endgerät (z. B. einem handelsüblichen Android-Smartphone) nötige Hardware, extrem klein zu bauen. Im Idealfall kann bei der Sensoreinrichtung des Geräts auf Bedienelemente und eine Anzeigeeinheit verzichtet werden.

Mittels einer auf dem mobilen Endgerät laufenden Software (Anwendungsprogramm) wird unter anderem das über die Elektroden gewonnene EKG ausgewertet und entschieden, ob ein Schock abgegeben werden soll. Sofern dies angebracht ist, wird die Defibrillation von der Software in die Anleitung des Ersthelfers integriert. Das EKG sowie die Daten zum abgegebenen Defibrillationsschock können gespeichert werden und an die medizinische Einrichtung übertragen werden, die den Patienten weiterversorgt.

Die Erfindung kann weiter derart ausgebildet sein, dass sie eine Aktivierungsvorrichtung umfasst, mittels welcher die Sensoreinrichtung eingeschaltet wird, d. h. zumindest der Mikroprozessor und der Hochspannungsspeicher mit der Batterie verbunden werden. Diese Aktivierungsvorrichtung kann ein manuell zu betätigender Kipp- oder Druckschalter sein.

Die Sensoreinrichtung kann weiterhin eine Positionierungshilfsvorrichtung in Form eines auszieh- oder ausklappbaren Bandes von vorgegebener Länge umfassen, sodass nach Anlegen der ausgezogenen/ausgeklappten Positionierungshilfsvorrichtung am Brustbein die Sensoreinrichtung an der für die Herzdruck-Massagen optimalen Position auf dem Brustkorb liegt.

Es kann vorgesehen sein, dass die Positionierungshilfsvorrichtung mit der Aktivierungsvorrichtung gekoppelt ist oder diese selbst darstellt, sodass mittel Betätigens der Positionierungshilfsvorrichtung zugleich die Sensoreinrichtung aktivierbar ist.

Alternativ kann vorgesehen sein, dass die Aktivierungsvorrichtung ein innerhalb des Gehäuses der Sensoreinrichtung angeordneter, druck- oder bewegungsempfindlicher Aktivierungsschalter ist, der die Sensoreinrichtung aktiviert, sobald der Ersthelfer Druck darauf ausübt.

Gemäß einer bevorzugten Ausgestaltung ist die Schnittstelle der Sensoreinrichtung zur Kommunikation mit einem mobilen Endgerät eine Drahtlos-Schnittstelle, wobei diese gemäß eines Bluetooth-Standards ausgelegt sein kann.

Weiterhin kann die Sensoreinrichtung einen Druck- bzw. Kraftsensor aufweisen, welcher beispielsweise eine vollständige Entlastung des Brustkorbes während der Herzdruckmassagen detektiert und/oder - wie bereits ausgeführt - als Aktivierungsvorrichtung für die Sensoreinrichtung eingesetzt ist.

Gemäß einer Ausgestaltung weist die Sensoreinrichtung zusätzlich einen Temperatur- und/oder Multi-Sensor zur Messung von medizinischen Parametern (z.B. Impedanz des Körpers) auf. Auch ein Sensor zur Prüfung der Atmung sowie der Qualität der Beatmung kann vorgesehen sein.

Alternativ ist das Haftmittel als eine adhäsive Beschichtung eines Oberflächenbereichs der Sensoreinrichtung ausgeführt, beispielsweise als rückseitiges Klebepflaster (Heftpflaster). In diesem Fall ist die Klebefläche des Pflasters mit einer abziehbaren Schutzfolie abgedeckt.

Es ist vorgesehen, dass die Batterie und der Hochspannungsspeicher derart ausgelegt sind, dass genau ein Defibrillationsschock abgebbar ist, wobei die nach Abgabe des Schocks in der Batterie verbleibende Restenergie lediglich dazu ausreicht, die Sensoren, den Mikroprozessor und die Schnittstelle für noch etwa 30 Minuten zu betreiben.

Auf der Außenseite seines Gehäuses kann die Sensoreinrichtung zumindest auf einem Bereich der Oberfläche, der - bei bestimmungsgemäßen Gebrauch der Sensoreinrichtung während der Reanimation - mit z. B. der vermittels der Sensoreinrichtung auf den Brustkorb des Patienten drückenden Hand des Reanimators in Kontakt tritt, eine Klebefläche in Form einer adhäsiven Beschichtung aufweisen. D.h., die Sensoreinrichtung kann neben dem auf seiner "Unterseite" angebrachten Haftmittel einen zusätzlichen, adhäsiv wirkenden Oberflächenbereich (Klebefläche) auf seiner "Oberseite" besitzen.

Der Speicher für elektrische Energie kann eine Einweg-Batterie oder eine wiederaufladbare Batteriezelle sein, wobei das Laden der Batterie induktiv erfolgen kann, d. h. die Sensoreinrichtung umfasst in diesem Fall zusätzlich eine induktive Lade-Schnittstelle zur Ankopplung an eine induktive Ladevorrichtung.

Gemäß einer Ausführungsform ist der Bewegungssensor der Sensoreinrichtung ein Beschleunigungssensor, beispielsweise ein Drei-Achs-Beschleunigungssensor, wobei aus den von dem Sensor erfassten Beschleunigungswerten sowohl die Eindrucktiefe wie auch die Kompressionsfrequenz errechnet werden können. Auch kann vorgesehen sein, dass die Sensoreinrichtung zwei, insbesondere redundante, Bewegungssensoren umfasst.

Die Positionierungshilfsvorrichtung kann in Form einer Maßverkörperung, z. B. eines Bandes, einer Schnur oder eines Stabes einer vorgegebenen Länge, ausgebildet sein, die aus dem Gehäuse herausklapp- oder herausziehbar ist. Vorzugsweise ist die Positionierungshilfsvorrichtung eine herausziehbare, z. B. abrollbare, flexible Maßverkörperung, deren maximale Länge 8 cm, vorzugsweise 5 cm, nicht übersteigt.

Es kann vorgesehen sein, dass die Maßverkörperung Markierungspositionen - z. B. ausgebildet als Einrastpunkte, die jeweils eine vorgegebene Ausziehlänge der Maßverkörperung beim Herausziehen aus dem Gehäuse der Sensoreinrichtung definieren - für Patienten unterschiedlicher Körpergröße aufweist, z. B. in Form einer Markierung für Kinder, für Jugendliche und für erwachsene Männer oder Frauen. Somit ist mittels Anlegens der Maßverkörperung an das Brustbein eine exakte Platzierung der Sensoreinrichtung auf dem Brustkorb des Patienten ermöglicht.

Insbesondere kann die Erfindung derart ausgestaltet sein, dass die Maßverkörperung aus einem flexiblen oder starren faserverstärktem Kunststoff, z. B. carbonfaserverstärkter Kunststoff (CFK) oder aus Aramid, besteht, wobei dessen temperaturabhängiger Längenausdehnungskoeffizient im Wesentlichen Null betragen kann.

Alternativ oder zusätzlich kann vorgesehen sein, dass die Maßverkörperung eine einseitig, zumindest partiell aufgebrachte Klebeschicht aufweist, sodass bei Betätigen der Positionierungshilfsvorrichtung die Maßverkörperung auf dem Brustkorb des Patienten mittels der Klebeschicht fixierbar ist.

Das Gerät zur Unterstützung eines Ersthelfers bei einer Herz-Lungen-Wiederbelebung wird nachfolgend anhand der Figuren näher erläutert, wobei gleiche oder ähnliche Merkmale mit gleichen Bezugszeichen versehen sind.

Dazu zeigen in schematischer Darstellung die
Fig. 1: eine Ausführung des Gerätes in Draufsicht; und
Fig. 2: eine Anwendung des Gerätes am Patienten.

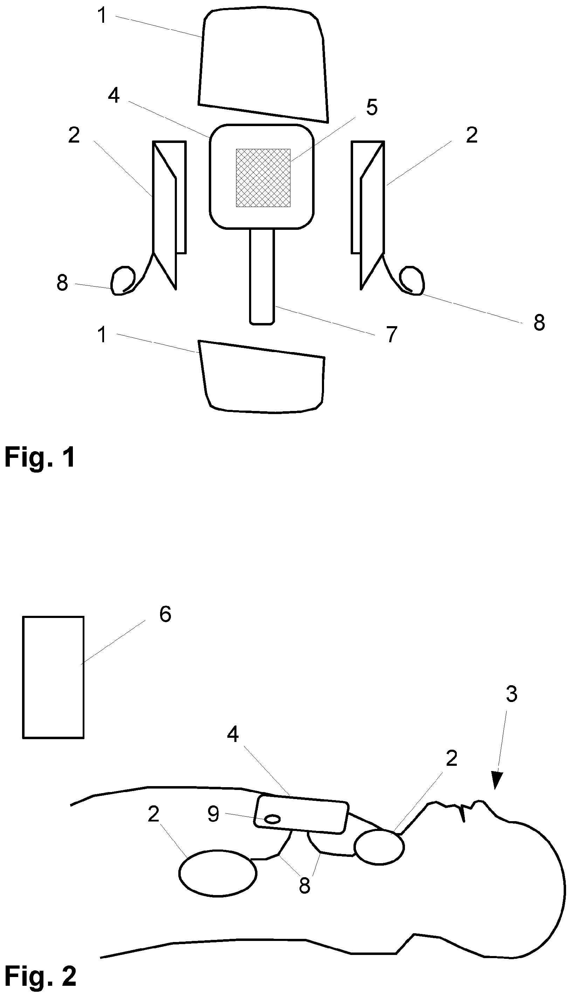

Gemäß Fig. 1 umfasst das Gerät das zweiteilige Transportgehäuse 1, das hier als wasserdichter Behälter ausgelegt ist. In demselben befinden sich die beiden Klebeelektroden 2 mit den Kabeln 8 und die Sensoreinrichtung 4. In Fig. 1 ist die aus der Sensoreinrichtung 4 bereits herausgezogene Positionierungshilfsvorrichtung 7 dargestellt. Rückseitig auf der Sensoreinrichtung 4 ist das Haftmittel 5 aufgebracht, mittels dessen die Sensoreinrichtung 4 auf dem Brustkorb befestigbar ist.

Gemäß Fig. 2 werden bei Eintreten des Notfalls die Sensoreinrichtung 4 und die Klebeelektroden 2 aus dem Transportgehäuse 1 entnommen. Die Klebeelektroden 2 werden auf dem Brustkorb des Patienten 3 befestigt und mittels der Kabel 8 an die Sensoreinrichtung 4 angeschlossen. Die Sensoreinrichtung 4 (hier eine Ausführung ohne Positionierungshilfsvorrichtung) wird durch Betätigen des Schalters 9 eingeschaltet, wodurch auch der Hochspannungsspeicher (nicht dargestellt) geladen wird, und anschließend auf dem Brustkorb des Patienten 3 befestigt.

Das mobile Endgerät 6, hier ein Smartphone, kommuniziert mittels Drahtlos-Schnittstelle (hier nach dem WLAN 802.11ax Standard) mit der Sensoreinrichtung 4. Eine auf dem mobilen Endgerät 6 gestartete Software leitet den Ersthelfer (nicht dargestellt) an, wobei sie bei Vorliegen der entsprechenden Indikation automatisiert - nachdem der Ersthelfer informiert wurde - einen einzelnen Defibrillationsschock auslöst.

### Liste der verwendeten Bezugszeichen

- 1: Transportgehäuse
- 2: Klebeelektrode
- 3: Patient
- 4: Sensoreinrichtung
- 5: Haftmittel
- 6: mobiles Endgerät
- 7: Positionierungshilfsvorrichtung
- 8: Kabel
- 9: Einschalter

## Patentansprüche

1. Gerät zur Unterstützung eines Ersthelfers bei einer Herz-Lungen-Wiederbelebung, umfassend eine Sensoreinrichtung (4) mit
- einer Oberseite, die im bestimmungsgemäßen Gebrauch der Sensoreinrichtung (4) eine Hand des Ersthelfers kontaktiert, und einer der Oberseite gegenüberliegenden Unterseite, die ein auf einem Oberflächenbereich angeordnetes Haftungsmittel (5) zur Befestigung der Sensoreinrichtung (4) auf dem Brustkorb eines Patienten (3) oder einer Übungspuppe aufweist,
- einem Bewegungssensor und
- einer Schnittstelle zum Senden und/oder Empfangen von Daten und/oder Befehlen,
wobei das Gerät ferner mindestens zwei mit der Sensoreinrichtung (4) verbindbare oder verbundene Klebeelektroden (2) aufweist,
wobei
- die Sensoreinrichtung (4) einen Speicher für elektrische Energie, einen Mikroprozessor und einen Hochspannungsspeicher mit Anschlüssen für die Klebeelektroden (2) aufweist, und
- die Sensoreinrichtung (4) eine Aktivierungsvorrichtung zum Verbinden zumindest des Mikroprozessors und des Hochspannungsspeichers mit dem Speicher für elektrische Energie aufweist,
**dadurch gekennzeichnet, dass**
- das Gerät weiterhin ein weitestgehend flaches, zumindest spritzgeschütztes Transportgehäuse (1) aufweist, in welchem die Sensoreinrichtung (4) und die Klebeelektroden (2) verstaubar sind, wobei das Transportgehäuse (1) geometrische Außenabmessungen von maximal 10 cm x 10 cm x 3 cm aufweist,
- die Klebeelektroden (2) faltbar sind, wobei sie Knickfalze zum Zusammenfalten während der Lagerung aufweisen, und
- der Speicher für elektrische Energie und der Hochspannungsspeicher derart ausgelegt sind, dass genau ein Defibrillationsschock abgebbar ist.

2. Gerät nach Anspruch 1, **dadurch gekennzeichnet, dass** zumindest ein Oberflächenbereich der Oberseite der Sensoreinrichtung (4) eine adhäsive Beschichtung besitzt oder aus einem adhäsiven Material besteht.

3. Gerät nach einem der vorstehenden Ansprüche, **dadurch gekennzeichnet, dass** die Schnittstelle drahtlos ist.

4. Gerät nach einem der vorstehenden Ansprüche, **dadurch gekennzeichnet, dass** der Speicher für elektrische Energie wiederaufladbar ist, wobei die Sensoreinrichtung (4) eine induktive Lade-Schnittstelle für eine induktive Ladevorrichtung aufweist.

5. Gerät nach einem der vorstehenden Ansprüche, **dadurch gekennzeichnet, dass** der Bewegungssensor ein Beschleunigungssensor ist.

6. Gerät nach einem der vorstehenden Ansprüche, **dadurch gekennzeichnet, dass** das Haftmittel (5) ein Heftpflaster ist, dessen Klebefläche mit einer abziehbaren Schutzfolie abgedeckt ist.

7. Gerät nach einem der vorstehenden Ansprüche, **dadurch gekennzeichnet, dass** es eine mit der Sensoreinrichtung (4) verbundene Positionierungshilfsvorrichtung (7) zur exakten Positionierung der Sensoreinrichtung (4) auf dem Brustkorb aufweist.

8. Gerät nach Anspruch 7, **dadurch gekennzeichnet, dass** die Positionierungshilfsvorrichtung (7) eine aus der Sensoreinrichtung (1) herausklapp- oder herausziehbare Maßverkörperung ist.

## Claims

1. An apparatus for supporting a first aider in a cardiopulmonary resuscitation, comprising a sensor device (4) with
- a top side that contacts a hand of the first aider when the sensor device (4) is used as intended, and a bottom side opposite the top side, which has an adhesive means (5) arranged on a surface area for fastening the sensor device (4) on the chest of a patient (3) or a training manikin,
- a movement sensor and
- an interface for sending and/or receiving data and/or commands,
wherein the apparatus further has two adhesive electrodes (2) connectable or connected with the sensor device (4),
wherein
- the sensor device (4) has a storage for electrical energy, a microprocessor and a high-voltage storage with terminals for the adhesive electrodes (2), and
- the sensor device (4) has an activation apparatus for connecting at least the microprocessor and the high-voltage storage to the storage for electrical energy,
**characterised in that**
- the apparatus furthermore has a largely flat, at least splash-proof transport housing (1) in which the sensor device (4) and the two adhesive electrodes (2) are storable, wherein the transport housing (1) has geometric external dimensions of at most 10 cm x 10 cm x 3 cm,
- the adhesive electrodes (2) are foldable, having kinked folds for folding up during storage, and
- the storage for electrical energy and the high-voltage storage are designed in such a way that exactly one defibrillation shock can be delivered.

2. The apparatus according to claim 1, **characterised in that** at least one surface area of the top side of the sensor device (4) has an adhesive coating or consists of an adhesive material.

3. The apparatus according to any one of the preceding claims, **characterised in that** the interface is wireless.

4. The apparatus according to any one of the preceding claims, **characterised in that** the storage for electrical energy is rechargeable, wherein the sensor device (4) has an inductive charging interface for an inductive charging device.

5. The apparatus according to any one of the preceding claims, **characterised in that** the motion sensor is an acceleration sensor.

6. The apparatus according to any one of the preceding claims, **characterised in that** the adhesive (5) is an adhesive bandage, the adhesive surface of which is covered with a removable protective film.

7. The apparatus according to any one of the preceding claims, **characterised in that** it has a positioning tool (7) connected to the sensor device (4) for the exact positioning of the sensor device (4) on the chest.

8. The apparatus according to claim 7, **characterised in that** the positioning tool (7) is a measuring standard that can be folded-out or pulled-out of the sensor device (1).

## Revendications

1. Appareil pour assister un secouriste lors d'une réanimation cardio-pulmonaire, comprenant un dispositif de détection (4) avec
- une face supérieure qui, lors de l'utilisation conforme du dispositif de détection (4), est en contact avec une main du secouriste, et une face inférieure opposée à la face supérieure, qui présente un moyen adhésif (5) disposé sur une zone de surface pour la fixation du dispositif de détection (4) sur le buste d'un patient (3) ou d'un mannequin d'exercice,
- un capteur de mouvement et
- une interface pour l'envoi et/ou la réception de données et/ou de commandes, l'appareil comprenant en outre au moins deux électrodes adhésives (2) pouvant être reliées ou reliées au dispositif de détection (4), dans lequel
- le dispositif de détection (4) comprend une mémoire pour l'énergie électrique, un microprocesseur et une mémoire haute tension avec des connexions pour les électrodes adhésives (2), et
- le dispositif de détection (4) comprend un dispositif d'activation pour connecter au moins le microprocesseur et l'accumulateur de haute tension à l'accumulateur d'énergie électrique,
**caractérisé en ce que**
- l'appareil présente en outre un boîtier de transport (1) largement plat, au moins protégé contre les projections, dans lequel le dispositif de détection (4) et les électrodes adhésives (2) peuvent être rangés, le boîtier de transport (1) présentant des dimensions extérieures géométriques de 10 cm x 10 cm x 3 cm au maximum,
- les électrodes adhésives (2) sont pliables, avec des plis pour les replier pendant le stockage, et
- l'accumulateur d'énergie électrique et l'accumulateur haute tension sont conçus de telle sorte qu'exactement un choc de défibrillation est délivrable.

2. Appareil selon la revendication 1, **caractérisé en ce qu'**au moins une zone de surface de la face supérieure du dispositif de détection (4) possède un revêtement adhésif ou est constituée d'un matériau adhésif.

3. Appareil selon l'une des revendications précédentes, **caractérisé en ce que** l'interface est sans fil.

4. Appareil selon l'une des revendications précédentes, **caractérisé en ce que** l'accumulateur d'énergie électrique est rechargeable, le dispositif de détection (4) comportant une interface de charge inductive pour un dispositif de charge inductive.

5. Appareil selon l'une des revendications précédentes, **caractérisé en ce que** le capteur de mouvement est un capteur d'accélération.

6. Appareil selon l'une des revendications précédentes, **caractérisé en ce que** le moyen adhésif (5) est un pansement adhésif dont la surface adhésive est recouverte d'un film protecteur détachable.

7. Appareil selon l'une des revendications précédentes, **caractérisé en ce qu'**il comporte un dispositif d'aide au positionnement (7) relié au dispositif de détection (4) pour le positionnement exact du dispositif de détection (4) sur le torse.

8. Appareil selon la revendication 7, **caractérisé en ce que** le dispositif d'aide au positionnement (7) est une mesure matérialisée pouvant être rabattue ou extraite du dispositif de détection (1).
